# EUROPEAN PATENT APPLICATION

(11) **EP 1 092 439 A1**
(43) Date of publication of application: **18.04.2001**
(21) Application number: 99119980.3
(22) Date of filing: 13.10.1999
(51) Int. Cl.: A61K 39/00, A61K 35/14, C12N 5/08, A61P 35/00, A61P 31/00, A61P 37/00

(54) **Activation of antigen-specific T cells by virus/antigen-treated dendritic cells**

(71) Applicant: Ahlert, Thorsten Dr., 69121 Heidelberg (DE)
(72) Inventor: Ahlert, Thorsten Dr., 69121 Heidelberg (DE)
(74) Representative: Müller-Boré & Partner Patentanwälte

(57) **Abstract**

The present invention relates to a vaccine containing virus/antigen-treated dendritic cells (DC) which stimulate an immune response in a patient obtainable by the activation of antigen-specific T cells (TC) *in vivo,* to a composition containing activated TC which are activated by the vaccine *in vitro,* to a pharmaceutical composition containing the vaccine and/or the composition as well as to methods for their production.

## Description

The present invention relates to a vaccine containing virus/antigen-treated dendritic cells (DC) which stimulate an immune response in a patient obtainable by the activation of antigen-specific T cells (TC) *in vivo,* to a composition containing activated TC which are activated by the vaccine *in vitro,* to a pharmaceutical composition containing the vaccine and/or the composition as well as to methods for their production.

The immune system of cancer patients but also of patients suffering from chronic infectious diseases, autoimmune diseases, renal failure with the need of dialysis, or inherited immune dysfunctions works inefficient and needs external help. The causes for this immune deficiency may be the diseases themselves or externally induced defects including immunosuppression by conventional cancer therapies. Furthermore, the recognition of diseases like cancer or infections by the immune system may face obstacles such as weak or inefficient presentation of disease-specific antigen.

Thus, reconstitution of immunocompetence for specific antigens is an object of intense research. Cell therapy by transfer of *in vitro*-activated allogeneic or autologous antigen-specific T cells or *in vivo* induction of such cells by vaccination procedures are current approaches aiming to solve the above-mentioned problems of immune deficiency.

T cells are lymphocytes which are able to provide specific and nonspecific immunologic help for several immune mechanisms and which may also directly attack and eradicate foreign or disease-associated cellular antigens. They are able to develop and/or transfer immunologic memory over months and years towards such antigens and thus, are important mediators of long-term immunologic protection.

The first approaches developed to confer an improved competence to a patient suffering from immune deficiency were adoptive cell transfer therapies. These therapies were mainly used for the treatment of cancer and employed in the first instance the so-called lymphokine-activated killer cells (LAK) and later lymphokine-activated tumor-infiltrating lymphocytes (TIL) for autologous transfer. The term "autologous" means that the cells which were transferred originated from the patient him- or herself. The killer cells were generated from peripheral blood or from tumor tissue which was freshly obtained by operation. The obtained killer cells were cultivated and activated mainly in medium containing interleukin 2 (IL-2), a T cell growth factor [refs. 1.,2.]. Other attempts of adoptive autologous and allogeneic cell transfer include stimulation of T cells with tumor cells and/or antibodies or cytokines *in vitro* or *in vivo* before they are adoptively transferred to the recipient [refs. 3. to 7.]. The term "allogeneic" means that the transferred cells originated from a nonidentical individual.

The use of dendritic cells (DC) for the *in vivo* or *in vitro* activation of antigen (peptide)-specific T cells has been established in very recent years [refs. 8. to 13.]. DCs are "professional" antigen-presenting cells which can be more powerful antigen-specific activators of T cells than the antigen-bearing cells themselves. DC can be pulsed or loaded with antigens such as peptides or cell lysates, for example antigens derived from tumor cells. The DCs process the antigenic material and integrate the products into MHC class I and/or class II complexes which are able to present them to T cells. For a full activation of killer T cells, a presentation of processed material in both class I and II MHC complexes is necessary, although the killer cells themselves are only able to recognise a presentation via MHC class I. MHC class II is necessary for the additional activation of helper T cells. This finding has led to the addition of substitutes like Keyhole-limpet haemocyanin (KLH) which represent MHC class II integrateable helper antigens. Furthermore, such a substitute would be able to represent a neoantigen and thus, can serve as a tracer molecule [ref. 8.].

Newcastle Disease Virus (NDV) is an avian paramyxovirus which has been used for a long time in cancer therapy. This virus can be used directly for infection and lysis of cancer cells *in vitro* or *in vivo,* and it can be used for modification of tumor cells in vaccines in order to give rise to an improved adhesion of stimulatory cells to their target cells. NDV may also induce a broad range of costimulatory signals for T cell activation when it is used for modification in cellular vaccines [refs. 14. to 22.].

However, the above-mentioned approaches display several problems and disadvantages.

In case of the use of cytokines for the activation and expansion of immune cells *in vitro,* the methods which have been used previously have not shown acceptable clinical risk-benefit relations. This is mostly due to an unspecific activation of the whole immune system and a dependency of the transferred cells on *in vivo* cytokine substitution after the application to the patient. The resulting treatment of patients with high-dose cytokines is accompanied by significant side effects which have led to an abandonment of this approach. More sophisticated methods using an antigen-specific component or a T cell receptor trigger for *in vitro* activation of T cells in addition to low-dose cytokines yielded cells which showed only a limited activity. Usually, the activity of the cells is readily suppressed after transfer into the patient, or the immune cells do not find (i.e. do not migrate to) the targeted cells *in vivo.* Therefore, the immunologic memory generated in this way is only short-lived and, moreover, leads to early disease progression after occasional therapeutic success.

Until now, DCs have been used clinically for *in vivo* induction of antigen-specific immune responses. However, obstacles which prevented a successful therapy using DCs have been the generation and/or isolation of a sufficient amount or functionally active DCs for therapeutic application. The strategies which have been developed until now, have rarely considered the possibility of a tolerance induction by insufficiently pulsed or insufficiently differentiated DCs. Furthermore, the *in vivo* generation of T cell responses with DCs can be difficult in patients with a deficient immune system.

The *in vivo* oncolysis using NDV has turned out to be difficult because of an efficient inactivation of the virus by the patient's immune system and because of virus-resistant tumor cells in heterogeneous tumors. Virus-modified tumor cell vaccines which have been used until now for *in vivo* activation of antigen-specific T cells show only limited effects which are only observed in early cancer stages. Approaches using NDV face further obstacles such as immune suppressed or immune deficient patients, insufficiently active T cells because of an antigen overload in the vaccinated patient or because of inhibitory factors which are produced by the target cells for T cells. Furthermore, suboptimal antigen presentation on antigen-varying target (i.e., in this case, tumor) cells and on *in vivo* preexisting antigen-presenting cells can be the reason for the failure of T cell activation.

A further problem of virus-modified cell vaccines which have been developed until now is that they need a significant number of viable antigen-bearing cells in order to reach a sufficient efficiency. This has limited the use of virus-modified cell vaccines in clinical applications so far because in clinical situations a comparably large amount of raw material (mostly surgically resectable tumor material) is available which contains considerable amounts of dead cells.

Moreover, the necessity of malignant viable cells in virus-modified vaccines results in the need of irradiation of the cells for their inactivation before *in vivo* use. This inactivation method is effective, however, it is difficult to apply with respect to the controlled pharmaceutical production process [refs. 20., 23. to 25.].

Therefore, the technical problem underlying the present invention is to improve the *in vivo* and *in vitro* induction of highly active antigen-specific immune stimulators, the effect of which is especially mediated by T cells and T memory cells during therapeutic clinical use. This improvement includes the reduction of the possibility of tolerance induction by T cell activation efforts and, thus, increasing the safety of the procedure.

The solution of the above technical problem is achieved by providing the embodiments as characterized in the claims.

In particular, the present invention relates to a vaccine containing activated dendritic cells for the activation of T cells which perform and stimulate a specific immunoresponse in a patient, wherein the dendritic cells are activated by the method comprising the steps of
(a) preparing an antigen and optionally treating the antigen with a virus *in vitro,*
(b) preparing monocytes from the patient or a relative thereof,
(c) developing dendritic cells from the monocytes by incubation *in vitro,*
(d) coincubating the obtained dendritic cells with the virus-treated antigen obtained in step (a) *in vitro* and/or with the untreated antigen obtained in step (a) plus the virus *in vitro,*
wherein the virus is capable of improving the adhesion of the antigen to and the presentation of the antigen by the dendritic cells and which is capable of modulating the activation, maturation and cosignalling of the dendritic cells.

The term "vaccine" as used herein means a composition or formulation containing activated dendritic cells which are capable of stimulating an immune response in a patient against antigens. The activated dendritic cells of the above-defined vaccine are capable of activating T cells *in vivo* as well as *in vitro.* For stimulating T cells *in vivo,* the activated dendritic cells may be administered, e.g. intracutaneously, subcutaneously or intralymphatically, to the patient, and patient's T cells migrate to the administration locus where they are activated by the dendritic cells. The vaccine according to the present invention may also contain substances which are prepared using recombinant DNA technology. Preferably, the vaccine according to the present invention contains one or more other T cell activating agents acting additively or synergistically with the dendritic cells.

The term "antigen" comprises any structure which is capable of inducing an immune response in an organism either by itself or when coupled to a suitable carrier molecule or cell. Therefore, antigens according to the present invention include low molecular compounds which serve as haptens as well as whole cells such as tumor cells as well as the parts thereof such as polypeptides, oligopeptides derived therefrom, lipids such as glycolipids, polysaccharides and nucleic acids. Further antigens according to the present invention are viruses as well as their parts and any prokaryotic organism such as bacteria as well as eukaryotic organisms. According to a preferred embodiment of the above-defined vaccine, the antigen is prepared from the patient, however, as defined above, the antigen may as well be prepared from other organisms or may be synthetic.

Preferably, the antigen which may be virus-treated in step (a) such as virus-treated living tumor cells may be inactivated without the use of irradiation prior to the coincubation with the dendritic cells in step (d) of the above-defined method. Preferred methods for inactivation and lysis of living cells such as living tumor cells include, for example, freeze-thawing and ultrasonification. The use of methods apart from irradiation poses less problems to the pharmaceutical production process.

The use of bone marrow in addition or instead of blood as the source for T cells which are to be activated leads to an increase of the yield of highly active antigen-specific T cells, for example T memory cells, for the stimulation by coincubation with NDV-modified DCs.

Preferably, the antigen such as a cell, e.g. a tumor cell, may be further purified during the preparation from the patient, for example by immunobead techniques. These techniques comprise the use of small magnetic metal beads (e.g. from Dynal or Milteny) which are coupled to antibodies directed against contaminating components such as cells or other agents. After having bound to the antibody-coupled beads during an incubation step, the contaminations are removed from the vaccine, e.g. a suspension of the activated dendritic cells, by applying a magnetic field which draws the beads out of the suspension. Further, the cells may be cryoconservated after their preparation, e.g. from the patient, in step (a) above and may be thawed before or after treatment with the virus in step (a) and/or coincubation with the dendritic cells in step (d) above.

Preferably, the developing dendritic cells are also coincubated with the virus during the step of incubation *in vitro* (c).

According to a further preferred embodiment of the above-defined vaccine, the virus used is selected from the group consisting of paramyxoviruses such as newcastle disease virus (NDV) or mumps virus, vaccinia virus, myxovirus, herpesvirus, AIDS virus, human papillomavirus (HPV) and mouse mammary tumor virus (MMTV).

The vaccine according to the present invention comprises dendritic cells which are activated with a virus and an antigen which is preferably prepared from a patient having a significantly impaired immune system. Preferably, this impairment of a patient's immune system may be caused by chronic disorders such as cancer, infections, renal failure which has to be treated by dialysis, autoimmune diseases and/or inherited immune dysfunctions.

The term "patient" as used herein comprises humans as well as animals. The preferred patient is a human. The "relative" of the patient is a person or animal, respectively, being related by blood and/or genetically via HLA-type with the patient, i.e. the human or animal.

A further embodiment of the present invention relates to a composition containing activated T cells which are capable of performing and stimulating a specific immunoresponse in a patient, wherein the T cells are activated by the method comprising the steps of
(i) preparing T cells from the patient or relative thereof,
(ii) treating the T cells with the vaccine as defined above *in vitro.*

Preferably, the treatment of T cells with the vaccine according to the present invention in step (ii) above is carried out by coincubation in a low- or medium-dose cytokine-containing medium for a short time. More preferably, the culture medium contains not more than 6000 U/ml of cytokines, for example IL-2, and the cultivation in the low-dose cytokine-containing medium is not longer than seven days.

According to preferred embodiments of the vaccine and the coomposition according to the present invention at least part of the monocytes prepared in step (b) of the above-defined vaccine and at least part of the T cells prepared in step (i) of the above-defined composition may be derived from the patient's or relative's bone marrow or blood. Therefore, in contrast to prior art cell therapy vaccines, bone marrow may be used in the above-defined vaccine as a very efficient source of monocytes from which dendritic cells are developed and, furthermore, in the above-defined composition as a very efficient source of (memory) T cells which are obtained for *in vitro* activation with virus-treated DCs in addition to monocytes or T cells, respectively, from peripheral blood.

In addition to the above-mentioned advantages of the vaccine and the composition according to the present invention, they show several further advantages due to the novel approach for the activation of T cells.

1,5x10⁶ NDV-modified DCs and an equivalent of 1,5x10⁶ target cells (for example tumor cells) when used as the antigen are needed for human *in vivo* vaccination or for a reasonably effective *in vitro* stimulation of T cells derived, for example, from humans. Taking these considerations into account the method for T cell activation as described above provides the possibility to use target cells as antigens which may be either dead or alive, since the uptake and processing of the material by the DCs leads to an antigen presentation to living TCs in the end. In contrast, in conventional virus-modified tumor cell vaccines at least 1,5x10⁶ target cells must be alive in order to lead to an efficient T cell activation and no more than 66% of dead cells should contaminate the living target cells [refs. 20., 24. to 26.]. However, an average of 50% of the target cells are dead in, for example, fresh tumor cells suspensions after cryoconservation which is mostly necessary for the storage of the raw material. Therefore, the use of NDV-modified DCs instead of original antigen-bearing living target cells reduces about 50% of the amount of raw material needed, since dead target cells as well as living target cells can be used as the antigen (the living target cells are preferably disintegrated by shock freezing or by the infection with the virus).

Furthermore, the use of a virus, for example NDV, in order to increase the DC number and to enhance their function facilitates and increases the generation of efficient DCs which can be used for *in vitro* or *in vivo* activation of (memory) T cells.

The use of a virus, for example a paramyxovirus such as NDV, which is capable of improving the adhesion of the antigen to the dendritic cells and which is capable of stimulating the activation of the dendritic cells as described above further reduces the probability of an induction of tolerance by an inefficient number and/or function of DCs in the patient. This advantage and the fact that the use of a virus as described above for increasing the number of DCs as well as their function improves and increases the generation of efficient DCs represent further surprising properties of the vaccine according to the present invention which can not be predicted from known properties of viruses such as NDV in tumor cell modification. Generally, DCs *per se* should be able to perform an optimal antigen presentation function and a costimulatory signalling for T cell activation. Therefore, in theory, there is no obvious need for the effects of a virus like NDV on DCs. However, while a virus such as NDV in fact induces a secretion of costimulatory cytokines and provides adhesion molecules for longer T cell-target cell interaction for the preparation of cellular vaccines such as the above described tumor cell vaccines, it improves the maturation and/or differentiation and/or antigen presentation, respectively, of DCs. Furthermore, the virus such as NDV could be able to modify instead of inducing a costimulatory signalling in DCs in order to generate an improved T cell activation. These effects on DCs were not expected from the known properties of viruses such as NDV on tumor cells and tumor cell vaccines. On the contrary, one would have expected from prior art studies that viruses suppress DCs. For example, Jenne et al. [ref. 27.] found a suppression of T cell stimulation properties by viruses and Raftery et al. [ref. 28.] found changes in DC function which were believed to contribute to human cytomegalovirus-associated immunosuppression after infection of DC with human cytomegalovirus.

Furthermore, the T cells which are contained in the composition according to the present invention and which are activated by the above-described method using the above-defined vaccine *in vitro,* exhibit a high efficiency and reduced dependency on *in vivo* application of cytokines which reduces potential side-effects of a therapy using the composition according to the present invention. Moreover, the T cells activated by the method as described above show a reduced sensitivity to inactivating mechanisms in a patient, since the T cells activated according to the present invention are more differentiated and more efficiently activated.

A further embodiment of the present invention relates to a pharmaceutical composition containing a pharmaceutically effective amount of the vaccine and/or the composition according to the present invention, optionally in combination with a pharmaceutically acceptable carrier and/or diluent, preferably for the curative or prophylactic treatment of cancer, infections and autoimmune diseases. Furthermore, the pharmaceutical composition according to the present invention may contain one or more other T cell activating agents which may act additively or synergistically with the vaccine and/or composition according to the present invention. The pharmaceutical composition according to the present invention may be applied by any conventional application route used in vaccination or cell therapy such as intravenous, intramuscular, intracutaneous, subcutaneous and/or intralymphatic administration, for example by infusion or injection.

Further embodiments of the present invention relate to methods for the preparation of activated dencritic cells and activated T cells, respectively. The method for the preparation of activated dendritic cells according to the present invention comprises the steps of
(a) preparing an antigen and optionally treating the antigen with a virus *in vitro,*
(b) preparing monocytes from the patient or a relative thereof,
(c) developing dendritic cells from the monocytes by incubation *in vitro,*
(d) coincubating the obtained dendritic cells with the virus-treated antigen obtained in step (a) *in vitro* and/or with the untreated antigen obtained in step (a) plus the virus *in vitro,*
wherein the virus is capable of improving the adhesion of the antigen to and the presentation of the antigen by the dendritic cells and which is capable of modulating the activation, maturation and cosignalling of the dendritic cells.

The method for the prepration of activated T cells according to the present invention comprises the steps of
(i) preparing T cells from a patient or relative thereof,
(ii) treating the T cells with the above-defined vaccine *in vitro.*

The present invention will be further illustrated by the following non-limiting example.

### EXAMPLE

***Adoptive cell therapy with allogeneic TCs which have been activated in vitro with NDV-modified DCs loaded with tumor cell material.***

### Preparation of the antigen from the patient

The preparation of NDV-modified tumor cells comprises the following steps:
(1) Isolation of tumor material by surgical intervention
(2) Dissociation of tumor cell material into a suspension of single cells by mechanical and enzymatic means: four times incubation for 30 min at 37°C with collagenase (5 U/ml) and DNase (15 U/ml). Optionally, immunobead purification of tumor cells which reduces contaminating non-tumor cells.
(3) Cryoconservation of tumor cells
(4) Thawing of tumor cells and modification/infection with NDV: 20 to 100 hemagglutinating units of virus per 1x10⁷ cells.
(5) Inactivation of NDV-modified tumor cells by 4 to 5 cycles of shock freezing and thawing.

### Preparation of T cells and dentritic cells from a relative of the patient

(1) Taking bone marrow and/or blood from the relative
(2) Preparing monocytes from the bone marrow and/or blood and inducing maturation and differentiation into DCs by standard cultivation with interleukin-4 (IL-4), granulocyte macrophage colony stimulating factor (GM-CSF) and tumor necrosis factor (TNF) with and without NDV
   (i) isolation of cells from 150 ml blood
   (ii) cultivation of monocytes for one day in RPMI 1640 plus 2 mM glutamine, 100 U/ml penicillin, 100 µg/ml streptomycin and supplemented with 5% autologous, noninactivated plasma, 1000 U/ml IL-4 (from Promocell) and 1000 U/ml GM-CSF
   (iii) change of medium and cultivation for two days in the above medium (day 1)
   (iv) change of medium, addition of TNFα (from Promocell) at a final concentration of 10 ng/ml (day 4)
(3) Loading/Pulsing of DCs with virus-modified tumor cell lysate by adding the lysate to the DCs followed by incubation in cell culture medium
   (v) addition of DCs to tumor cells at a ratio of 1 part DCs to 3 parts dead tumor cells in 1 ml X-vivo medium, centrifugation at low speed [1000 revolutions per hour (rph)], incubation at 37°C for 4 h (day 5)
   (vi) control of antigen-loaded DCs using fluorescence-activated cell sorting (FACS) analysis
   (vii) cultivation of antigen-loaded DCs in RPMI + 5 % plasma + IL-4 + GM-CSF + TNFα for three days (until day 8)
(4) Isolation of TCs from bone marrow and/or blood by a method comprising an immunobead enrichment step (this may be carried out during the generation/loading phase of the TCs)
   (viii) isolation of TCs from 150 ml blood (erythroycte lysis, adherence, Pan T cell isolation kit)
   (ix) control of TCs using FACS analysis
(5) Expansion of TCs in cell culture
(6) Coincubation of TCs and DCs which have been pulsed with NDV-modified tumor lysate which comprises a short incubation in the presence of cytokines at low- or medium-dose in order to avoid induction of dependency of the TCs on these cytokines
   (x) addition of TCs to the antigen-loaded DCs in a ratio of 1 part DCs to 34 parts TCs and incubation in fresh RPMI supplemented with 5% plasma (but without cytokines) for three days (until day 11)
   (xi) change of medium and addition of IL-2 (6000 U/ml) on day 11 and cultivation for three days
   (xii) harvesting the activated TCs, resuspending the TCs in 10 ml medium I, filling the suspension in a syringe for intravenous injection, filtration; the filtrate is taken up in 400 µl medium I and injected subcutaneously.
(7) Analysis of antitumor activity of the activated TCs using ELISPOT T cells are coincubated (challenged) with antigen for 20 h. Thereafter, γ-interferon (IFN-γ) production is detected for each single T cell on a plate coated with anti-IFN-γ antibodies. Bound IFN-γ is detected in spots surrounding the T cells by means of ELISA stain.

### Therapy

(1) Day 1 to 14
   Immunosuppression of the patient with medium-dose or dose-intensified chemotherapy and/or radiotherapy and/or corticosteroids and cyclosporin, which is necessary in order to avoid the rejection of the allogeneic DC-NDV tumor-actived TCs of the patient's relative.
   The patient is treated with 80 mg/m² taxol, 40 mg/m² epirubicin and 50 mg of hydrocortisol per day for two consecutive weeks. In addition, 30 Gray irradiations of a bone metastasis were carried out.
(2) Day 15
   Intravenous infusion of the allogeneic TCs which were activated by NDV-DC treatment
   About 5x10⁸ T cells which have been activated by the above-described method are infused in a volume of 250 ml Ringer lactate solution.
(3) Next cycle of treatment after a break of six weeks.

### Materials

recombinant human (rHU) IL-4cc dissolved in phosphate buffered saline (PBS)/1 % human serum albumin (HSA) (stock solution: 1x10⁵ U/ml, corresponding to 1x10⁵ µg/ml)
GM-CSF dissolved in PBS/1% bovine serum albumin (BSA) (stock solution: 1x10⁵ U/ml)
rHU TNFα dissolved in RPMI 1640/1% BSA (stock solution: 1 µg/ml)
IL-2 dissolved in X-vivo (stock solution: 6x10⁵ U/ml, diluted 1:100), proleukin (from Chiron)

### References

1. Rosenberg SA, Lotze MT, Muul LM, Chang AE, Avis FP, Leitmann, Line-han WM, Robertson CN, Lee RE, Rubin JT, Seipp CA, Simpson RN, White DE: A progress report on the treatment of 157 patients with advanced cancer unsing lymphokine-activated killer cells and interleukin-2 or high-dose interleukin-2 alone. N Engl J Med 316. (15) 889 - 897, 1987
2. Rosenberg SA, Packard BS, Aeberseld PM, Solomon D, Topalian SL, Toy ST, simon P, Lotze MT, Vang JC, Seipp CA, Simpson C, carter C, Bork S, Schwarzentruber D, Wei JP, White DE: Use of tumorinfiltrating lymphocytes and interleukin-2 in the immunotherapy of patients with metastatic melanoma. New Engl J Med 319. 1676 - 1680, 1988
3. Fichtner K-P, Schirrmacher V, Griesbach A, Hull WE: In Vivo 1H-NMR microimaging with respiratory triggering for monitoring adoptive immuno therapiy of metastatic mouse lymphoma. Magnetic Resonance In Medicine (MRM) 38. 440 - 455, 1997
4. Yamagishi H, Ueda Y, Oka T: A case report of immunotherapy on a patient with advanced gastric cancer by adoptive transfer of OK-432 reactive HLA-matched allogeneic lymphocytes. Cancer Immunol Immunother 46.113-119,1998
5. Schirrmacher V, Beckhove P, Krüger A, Rocha M, Umanski V, Fichtner K-P, Hull WE, Zangemeister-Wittke U, Griesbach A, Jurianz K, Hoegen Pv: Effective immune rejection of advanced metastasized cancer. Int J Oncol 6. 505 - 521, 1995
6. Cardoso AA, Seamon MJ, Afonso HM, Ghia P, Boussiotis VA, Freeman GJ, Gribben JG, Sallan SE, Nadler LM: Ex vivo generation of human anti-pre-B leukemia-specicic autologous cytolytic T cells. Blood 90. (2) 549 - 561, 1997
7. Tani M, Tanimura H, Yamaue H, Mizobata S, Iwahashi M, Tsunoda T, Noguchi K, Tamai M, Hotta T, Terasawa H, Arii K: Generation of CD4+ cytotoxic T-lymphocytes stimulated by immobilized anti-CD3 monoclonal antibody and interleukin-2 in cancer patients. Int J Cancer 60. 802 - 807, 1995
8. Nestle FO, Alijagic S, Gilliet M, Sun Y, Grabbe S, Dummer R, Burg G, Schadendorf D: Vaccination of melanom patients with peptide- or tumor lysate-pulsed dendritic cells. Nature Medicine 4. (3) 328 - 332, 1998
9. Höltl L, Rieser C, Papesh C, Ramoner R, Herold M, Klocker H, Radmayr C, Stenzl A, Bartsch G, Thurnher M: Cellular and humoral immune responses in patients with metastatic renal cell carcinoma after vaccination with antigen pulsed dendritic cells. J Urol 161. 777 - 782, 1999
10. Hsu FJ, Benike C, Fagoni F, Liles TM, Czerwinski D, Taidi B, Engleman EG, Levy R: Vaccination of patients with B-cell lymphoma using autologous antigen-pulsed dendritic cells. Nature Medicine 2. 52 - 58, 1996
11. Salgaller ML, Lodge PA, McLean JG, Tjoa BA, Loftus DJ, Ragde H, Kenny GM, Rogers M, Boynton AL, Murphy GP: Report of immune monitoring of prostate cancer patients undergoing T cell therapy using dendritic cells pulsed with HSA-A2-specific peptides from prostate-specific membrane antigen (PSMA). Prostate 35. 144 - 148, 1998
12. Bennet SRM: Induction of a CD8- cytotoxic T lymphocyte response by crosspriming requires cognate CD4-help. J Exp Med 186. 65 - 70, 1997
13. Peters JH, Gieseler R, Thiele B, Steinbach F: Dendritic cells: from ontogenetic orphans to myelomonocytic descendants. Immunology today 17. (6) 273 - 278, 1996
14. Ahlert T and Schirrmacher V. Isolation of a human melanoma adapted Newcastle Disease Virus mutant with highly selektive replication patterns. canc res 1990;505962-8.
15. Ahlert T, Kaufmann M, Bastert G, Schirrmacher V. : Active Specific Immunotherapy (ASI) with virally modified autologous tumor cells in breast and ovarian cancer: factors influencing immunological vaccination success. J of Canc Res and Clin Oncol 116 / supplement. :80, 1990 (abstr)
16. Cassel WA, Murray DR: A ten-year follow-up on stage II malignang melanoma patients treated postsurgically with Neuwcastle disease virus oncolysate. Med Oncol & Tumor Pharmacother 9. 169 - 171, 1992
17. Cassel WA, Garret RE: Newcastle Disease Virus as an antineoplastic agent. Cancer 66. (7) 1517- 1523, 1965
18. Csatary LK, Eckhard S, Bukosza I, Czegledi F, Fenyvesi C, Gergely P, Bodey B, Csatary CM: Attenuated veterinary virus vaccine for the treatment of cancer. Cancer Detection and Prevention 17. 619 - 627, 1993
19. Schirrmacher V, Haas C, Bonifer R, Ahlert T, Gerhards R, Ertel C: Human tumor cell modification by virus infection: an efficient and safe way to produce cancer vaccine with pleiotropic immune stimulatory properties when using Newcastle disease virus. Gene therapy 6. 63 - 73, 1999
20. Schirrmacher V, Ahlert T, Pröbstle T, Steiner H-H, Herold-Mende C, Gerhards R, Hagmüller E: Immunisation with virus modified tumor cells. Seminars in oncology 25. (6) 677 - 696, 1998
21. Schirrmacher V, Hoegen von P, Heicappell R: Virus modified tumor cell vaccines for active specific immunotherapy of mecrometastases: Expansion and activation of tumor-specific T cells. Immunity to Cancer 2. 391 - 399, 1989
22. Schirrmacher V, Ahlert T, Heicappell R, Appelhans B, Hoegen von P: Successful application of non-oncogenic viruses for antimetastatic cancer immunotherapy. Cancer Res 5. 19 - 49, 1986
23. Ahlert T, Sauerbrei W, Bastert G, Ruhland S, Bartik B, Simiantonaki N, Schumacher J, Häcker B, Schuhmacher M, Schirrmacher V: Tumor-cell number and viabilitiy as quality and efficacy parameters of autologous virus-modified cancer vaccines in patients with breast or ovarian cancer - Errata. Journal of clinical oncology 15. (4) 2763, 1997
24. Schirrmacher V, Griesbach A, Zangemeister-Wittke U: y-irradiated viable tumor cells as whole-celle vaccines can stimulate in situ syngenelc antitumor cytotoxic T lymphocytes and delayed-type hypersensitivity reactivity whereas tumor cell lysates elicit only delayed-type hypersensitivity reactivity. Vaccine Res 3 - No.1. 31 - 48, 1994
25. Schirrmacher V, Hoegen von P: Importance of tumor cell membrane integrity and viability for cytotoxic T lymphocyte activation by cancer vaccines. Vaccine Res 2 - No. 3. 183 - 196, 1993
26. Ahlert T, Sauerbrei W, Bastert G, Ruhland S, Bartik B, Simiantonaki N, Schumacher J, Häcker B, Schumacher M, Schirrmacher V: Tumor cell number and viability as quality and efficacy parameters of autologous virus modified cancer vaccines. J Clin Oncol 15. 1354 - 1366, 1997
27. Jenne L et al., Immunobiology 200 (1999) 3-5, pp. 562
28. Raftery M et al., Immunobiology 200 (1999) 3-5, pp. 568

## Claims

1. A vaccine containing activated dendritic cells for the activation of T cells which perform and stimulate a specific immunoresponse in a patient, wherein the dendritic cells are activated by the method comprising the steps of
(a) preparing an antigen and optionally treating the antigen with a virus *in vitro,*
(b) preparing monocytes from the patient or a relative thereof,
(c) developing dendritic cells from the monocytes by incubation *in vitro,*
(d) coincubating the obtained dendritic cells with the virus-treated antigen obtained in step (a) *in vitro* and/or with the untreated antigen obtained in step (a) plus the virus *in vitro,*
wherein the virus is capable of improving the adhesion of the antigen to and the presentation of the antigen by the dendritic cells and which is capable of modulating the activation, maturation and cosignalling of the dendritic cells.

2. The vaccine according to claim 1, wherein the method further comprises the step of inactivating the virus-treated antigen obtained in step (a) without the use of irradiation.

3. The vaccine according to claim 2, wherein the virus-treated antigen is inactivated by freeze-thawing or ultrasonification.

4. The vaccine according to anyone of claims 1 to 3, wherein at least part of the monocytes are prepared from the patient's or relative's bone marrow.

5. The vaccine according to anyone of claims 1 to 4, wherein the antigen is prepared from the patient.

6. The vaccine according to anyone of claims 1 to 5, wherein the antigen is a cell or a part thereof.

7. The vaccine according to claim 6, wherein the cell is a tumor cell.

8. The vaccine according to claim 6 or 7, wherein the cell is further purified by immunobead techniques.

9. The vaccine according to anyone of claims 6 to 8, wherein the cells are cryoconservated after their preparation and thawed before or after the treatment with the virus in step (a) and/or the coincubation with the dendritic cells in step (d).

10. The vaccine according to anyone of claims 1 to 9, wherein in step (c) the developing dendritic cells are also coincubated with the virus.

11. The vaccine according to anyone of claims 1 to 10, wherein the virus is selected from the group consisting of paramyxoviruses, vaccinia virus, myxovirus, herpesvirus, AIDS virus, human papillomavirus and mouse mammary tumor virus.

12. The vaccine according to claim 11, wherein the paramyxovirus is newcastle disease virus or mumps virus.

13. The vaccine according to anyone of claims 1 to 12, wherein the patient's immune system is significantly impaired.

14. The vaccine according to claim 13, wherein the impairment of the immune system is caused by cancer, infections, renal failure, autoimmune diseases and/or inherited immunodysfunctions.

15. The vaccine according to anyone of claims 1 to 14, wherein the patient is a human.

16. The vaccine according to anyone of claims 1 to 15 further containing one or more other T cell activating agents.

17. A composition containing activated T cells which are capable of performing and stimulating a specific immunoresponse in a patient, wherein the T cells are activated by the method comprising the steps of
(i) preparing T cells from the patient or relative thereof,
(ii) treating the T cells with the vaccine according to anyone of claims 1 to 16 *in vitro.*

18. The composition according to claim 17, wherein at least part of the T cells are prepared from the patient's or relative's bone marrow.

19. The composition according to claim 17 or 18, wherein the treatment of the T cells is carried out in a culture medium containing not more than 6000 U/ml of IL-2.

20. A pharmaceutical composition containing a pharmaceutically effective amount of the vaccine according to anyone of claims 1 to 16 and/or the composition according to anyone of claims 17 to 19, optionally in combination with a pharmaceutically acceptable carrier and/or diluent.

21. The pharmaceutical composition according to claim 20 for the curative or prophylactic treatment of cancer, infections and autoimmune diseases.

22. A method for the preparation of activated dendritic cells comprising the steps of
(a) preparing an antigen and optionally treating the antigen with a virus *in vitro,*
(b) preparing monocytes from the patient or a relative thereof,
(c) developing dendritic cells from the monocytes by incubation *in vitro,*
(d) coincubating the obtained dendritic cells with the virus-treated antigen obtained in step (a) *in vitro* and/or with the untreated antigen obtained in step (a) plus the virus *in vitro,*
wherein the virus is capable of improving the adhesion of the antigen to and the presentation of the antigen by the dendritic cells and which is capable of modulating the activation, maturation and cosignalling of the dendritic cells.

23. A method for the prepration of activated T cells comprising the steps of
(i) preparing T cells from a patient or relative thereof,
(ii) treating the T cells with the vaccine according to anyone of claims 1 to 16 *in vitro.*
